Europäisches Patentamt

European Patent Office

Office européen des brevets

(19)

(11) Publication number: **0 476 674 A1**

## EUROPEAN PATENT APPLICATION

(12)

(21) Application number: **91115981.2**

(22) Date of filing: **19.09.91**

(51) Int. Cl.⁵: **G01N 1/26**, G01N 31/10

(30) Priority: **20.09.90 US 585626**

(43) Date of publication of application:
**25.03.92 Bulletin 92/13**

(84) Designated Contracting States:
**AT BE CH DE DK ES FR GB GR IT LI LU NL SE**

(71) Applicant: **SPACE BIOSPHERES VENTURE**
**Biosphere 2 Conference Center, Highway 77,**
**96.5 Mile Marker, P. O. Box 689**
**Oracle, Arizona 85623(US)**

(72) Inventor: **MacCullum, Taber K.**
**Highway 77, Mile Marker 96.5, P.O. Box 689**
**Oracle, Arizona 85623(US)**
Inventor: **Fitz, Dennis R.**
**2174 North Mills**
**Claremont,California 91711(US)**

(74) Representative: **Patentanwälte Grünecker,**
**Kinkeldey, Stockmair & Partner**
**Maximilianstrasse 58**
**W-8000 München 22(DE)**

(54) **Air sampling and analysis system.**

(57) An air sampling and analysis system has a plurality of air sampling tubes extending between an analytical laboratory and a number of remote locations where samples are taken at sample inlets (34). Means (51, 52) including three-way valves are provided for continually drawing air through the sampling tubes during times when they are not connected to a analytical instrument (2-8, 11,12) for measuring concentration of various gases. Means (44, 48) are also provided adjacent to the inlet of at least some of the sampling tubes for converting a vapor which may not travel the full length of the sampling tube, to a gas which will travel the full length of the tube. The concentration of the vapor can be found by measuring the composition of the air sample with conversion and subtracting a similar composition without conversion. Examples of vapors that are converted include hydrogen sulfide to sulfur dioxide and ammonia to nitrogen oxide.

EP 0 476 674 A1

## Background of the Invention

This invention relates to a system for sampling air at a plurality of remote locations and conveying the air to an analytical laboratory for analysis of the composition of the air.

There is being established near Oracle, Arizona, a completely closed ecological system referred to as Biosphere 2. The system completely encloses about one hectare of land and 175,000 cubic meters of space isolated from the earth's environment by an impermeable skin so that no matter is transferred. The above ground portion of the skin is largely transparent for receiving solar radiation. Electrical energy is provided to the closed system, and heat may be transferred to or from the system as required. Thus, the Biosphere 2 closed ecological system is closed as to matter, but open as to energy.

The Biosphere 2 system provides facilities for occupation by eight humans who can remain sealed in the system under healthful living conditions for two years or more. Diverse plant, animal and microorganism populations in the system assure balancing of the ecology for long periods.

The atmosphere within the closed system could drift far from the composition usual in outside air, or could accumulate toxic vapors which could be harmful to the inhabitants. Thus, it is desirable to provide means for controlling the cycles of matter in the closed ecological system and to do that one needs to know what is happening in the atmosphere for detecting trends toward imbalance so that remedial steps may be taken before the undesirable condition becomes extreme. Furthermore, knowledge of the composition of the atmosphere is important data for evaluating experiments conducted within the closed system.

Since conditions may vary considerably from place to place within the large closed system it is desirable to sample the air in various locations throughout Biosphere 2 for analysis. It is desirable to employ precision instruments maintained in a single analytical laboratory for analyses performed throughout the system. This introduces a requirement for gathering samples from remote locations and conveying them to the analytical laboratory.

As with any sampling system it is important that the air sample delivered to the analysis instruments should be representative of the air at the remote location. It is desirable that the sampling system be arranged so that the taking of samples and analysis may be automated and controlled by computer inputs. It is also desirable that means be provided for calibrating the system to assure that the analytical data are truly representative of the air being sampled.

## Summary of the Invention

There is therefore provided in practice of this invention an air analysis system having a plurality of air sampling tubes with sample inlets at remote locations and extending to an analytical laboratory or the like. Means are provided for continually drawing air through each sampling tube so that the air in the tube is continually representative of the air to be sampled. Means are also provided for selectively disconnecting the means for drawing air from any selected sampling tube and connecting the selected sampling tube with one or more analysis instruments. Preferably dryers are provided at each remote location for drying air drawn into each sampling tube.

Furthermore, there are vapors which may not travel the full length of the sampling tube because of absorption or reaction. Therefore, means are provided adjacent to the inlet of the tube for converting the vapor to a gas which will travel the full length of the sampling tube. One may then determine the amount of the vapor by comparing the concentration of the gas with and without conversion. Examples of such conversion include converting ammonia to nitric oxide and converting hydrogen sulfide to sulfur dioxide.

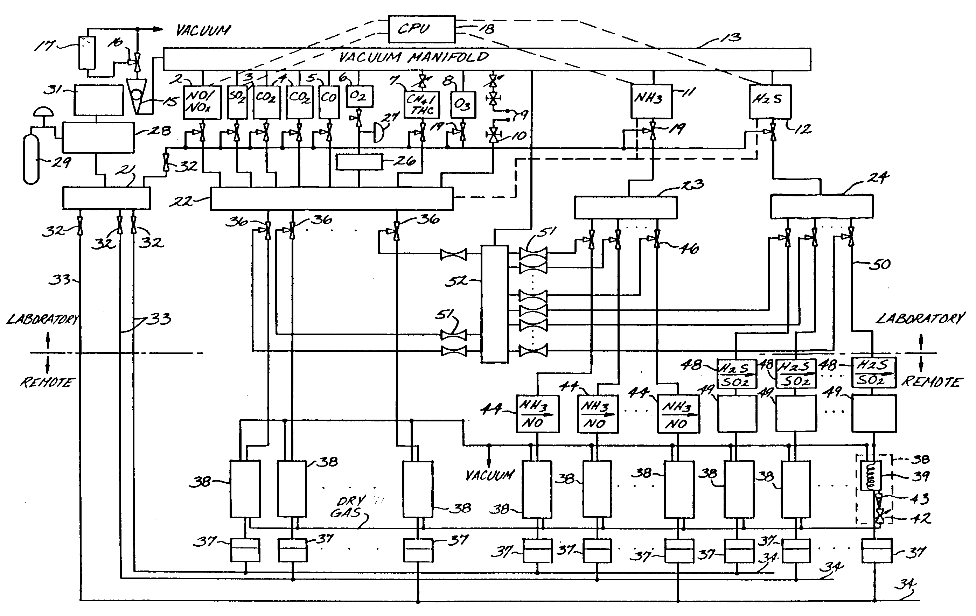

## Brief Description of the Drawing

These and other features and advantages of the present invention will be appreciated as the same becomes better understood by reference to the following detailed description, when considered in connection with the accompanying drawing, which is a schematic block diagram of an air sampling and analysis system constructed according to principles of this invention.

## Detailed Description

The air sampling and analysis system can be considered as having two major parts. An analytical laboratory, illustrated in the upper portion of the schematic drawing, and a portion at a plurality of remote locations where samples are taken, illustrated in the lower portion of the drawing. Inert plastic or metal tubing (e.g., PTFE) connects the remote locations with the laboratory. The remote sampling locations may be many tens of meters or even hundreds of meters from the laboratory.

A variety of conventional commercially available gas analysis instruments may be used in the analytical laboratory. It is desirable to employ instruments which can operate automatically without manual manipulation so that analyses can be performed intermittently according to a prearranged schedule, day or night.

In an exemplary embodiment these instruments include an analyzer 2 for the concentration of nitrogen oxides, $NO_x$, and particularly, nitric oxide. Another instrument 3 is used for measuring the concentration of sulfur dioxide. In the illustrated embodiment, two instruments 4 are used in parallel for measuring carbon dioxide concentration. One of the carbon dioxide instruments has a low sensitivity range and the other has a high sensitivity range so that relatively broad swings in carbon dioxide concentration may be monitored. The $CO_2$ measuring instruments are connected to the vacuum manifold by pumps (not shown) for minimizing pressure variations at the instruments. Another instrument 5 is used for measuring the carbon monoxide concentration in the atmosphere.

An analytical instrument 6 suitable for measuring oxygen concentration in air is also used. Still another instrument 7 is used for measuring methane concentration and total hydrocarbons (THC) other than methane. Ozone is monitored with another instrument 8. Additional connection ports 9 are provided between toggle valves 10 so that other instruments may be connected into the system as may be desired for measuring other gases.

An analysis instrument 11 labeled in the drawing for measuring ammonia is actually similar to the instrument 2 for measuring nitric oxide. As explained in greater detail hereinafter, the ammonia concentration is found by converting ammonia to nitric oxide (NO) and finding the difference between the NO concentrations with and without such conversion. Similarly, the laboratory includes an instrument 12 for determining hydrogen sulfide concentration. This instrument is the same as the instrument 3 for measuring sulfur dioxide concentration. The concentration of hydrogen sulfide is determined after catalytically converting hydrogen sulfide to sulfur dioxide. Means are provided for scrubbing all the $SO_2$ from the gas stream before converting the H2S to $SO_2$.

Each of the analytical instruments is connecting to a vacuum manifold 13 maintained at a low pressure of about 50 centimeters of mercury. For monitoring purposes, the vacuum manifold is connected to the vacuum source by a rotameter 14. A three-way solenoid operated valve 16 permits bypassing of gas from the vacuum manifold through a scrubber 17 before going to the vacuum pumps. This may be used when toxic or noxious fumes are present in the system, such as may occur due to deliberate additions during calibration.

The inlet for each of the analytical instruments is connected to a three-way solenoid operated valve 19. The system includes a substantial number of three-way solenoid operated valves, only a portion of which are marked with identifying numerals in the drawing. The same symbol is used for each of these valves. The three-way valves 19 permit selective connection of each of the instruments to a calibration manifold 21 or a principal sample manifold 22. The ammonia-measuring instrument 11 may be selectively connected to a subsidiary sample manifold 23. The hydrogen sulfide analytical instrument 12 may be selectively connected to another subsidiary sample manifold 24. These instruments may also be connected to the main sample manifold 22 if desired as indicated by a dashed line in the drawing.

The oxygen analysis instrument 6 is connected to the sample manifold by a pump 26 controlled by a pressure regulator 27. Such a system is used since the preferred instrument for measuring the relatively high oxygen concentration determines an absolute quantity of oxygen and is therefore rather sensitive to pressure variations. The relatively minor effects of pressure variations on the other analytical instruments can be handled by calibration or calculation. The three-way valve for the oxygen analytical instrument 6 is not connected to the calibration manifold, but instead is connected to an oxygen source (not shown) for calibration.

Each of the analytical instruments is connected to the central processing unit 18 of a computer which provides control for the system and also gathers data, not only from the instruments in the laboratory, but from other portions of the environment being analyzed. Only a fraction of these connections are indicated in the drawing by dashed line. The computer also controls the many solenoid valves in the system. This permits automatic sampling and analysis on a scheduled basis at an arbitrary number of remote locations. The same computer may also be used for data analyses, correcting for interferences, and activating alarms in the event a gas being sampled exceeds a preset limit which indicates that some corrective action may be desired.

The calibration manifold 21 is connected to a calibrator 28 which provides controlled mixing of trace quantities of gas from a calibration gas source 29 with air from a pure air source 31. The calibration gas may be obtained from a gas generator or a container of stored gas of a desired composition. Solenoid valves 32 permit selective connection of the calibration manifold with the analytical instruments or to any of a plurality of calibration tubes 33 leading to the remote sampling locations.

The gas sampling system may be used for taking and analyzing samples of air from any arbitrary number of remote locations. For example, in Biosphere 2, an air sample may be taken at each of six different biomes within the closed system. Each of the remote sampling stations may be similar where it is anticipated that generally similar conditions will prevail at each station. Clearly one could, if desired, have different arrangements at various sampling stations.

Because of such similarities, common features are indicated schematically in the drawing or may be omitted from most of the sampling stations.

Typically, each sampling station inlet 34 is normally open to permit continual air flow. When one calibrates the system, corresponding valves 32 at the calibration manifold are opened and calibration gas passed through the tubes 33. The quantity of gas introduced for calibration is greater than the amount drawn through the sample tubes for analyses. The excess gas is discharged through the open end of the sampling station inlet, thereby excluding air from the biome from entering the tube during calibration.

The sampling station inlets may also be used for temporarily connecting a permeation tube calibrator or the like (not shown), at a sampling location for calibration of the system, particularly for vapors such as ammonia or hydrogen sulfide which may not travel the full length of the calibration tubes 33 from the calibration manifold. One may also temporarily introduce nitrogen into the sampling station inlets for a sufficient time to be lethal to aerobic organisms that might populate the tubing and interfere with accurate analyses. This may be done by going to the inlet with a portable nitrogen supply, or may be done by introducing nitrogen via the calibration tubing 33 at a flow rate greater than drawn through the sample tubes.

Each of the sampling stations in this embodiment has three taps to which air samples are routed. A principal connection for each sample inlet is to the main sample manifold 22 which provides connections to the analytical instruments in the laboratory. Each of these connections to the main sample manifold is made by way of a three-way solenoid valve 36. These valves may be selectively opened so that each sample inlet is, in turn, connected to the main sample manifold for analysis of air drawn into the system through that respective sample inlet.

Each tap at the sample inlets has a filter 37 and dryer 38. The filter is simply to keep debris out of the sampling tubes. Each dryer comprises a conventional diffusion drying tube 39. Such a diffusion drying tube comprises a chamber containing a membrane permeable to water vapor. A dry vacuum is drawn on one side of the membrane and water vapor from the other side diffuses through the membrane for drying the gas sample. The vacuum side of each diffusion drying tube is connected to a source of dry compressed air 41 by way of a manual flow control valve 42 and rotameter 43 for controlling dry air flow through the vacuum side of the diffusion tube dryer.

The filter 37 is connected on the other side of the membrane so that the gas samples are dried at the sample locations and little, if any, water vapor travels through the sampling tubes. Diffusion tube dryers are desirable since they operate without periodic recharging or other maintenance. If desired, intermittent measurements of relative humidity may be made of the air continually drawn through each sampling station inlet to verify that the dryers are operating properly.

Another group of sample taps, one for each sample inlet, are connected to catalytic converters 44 which convert ammonia in the air to nitric oxide. Each of these catalytic converters is connected to one of the subsidiary sample manifolds 23 by a three-way solenoid valve 46. The catalytic converters are located adjacent to each sample inlet, upstream from long sampling tubes 47 leading to the subsidiary sample manifold. Ammonia in the air samples may react or absorb unpredictably in the long sampling tubes. Such unpredictability is avoided by converting the ammonia to nitric oxide which will travel the full length of the sampling tubes for analysis in the analytical laboratory.

Similarly, another set of sample taps, one for each sample inlet, is connected to scrubbers 49 for removing sulfur dioxide from the gas stream and catalytic converters 48 which convert hydrogen sulfide to sulfur dioxide. These converters are provided adjacent to each sample inlet since hydrogen sulfide is also a vapor that may travel unpredictably through long sampling tubes 50 leading to the other subsidiary sample manifold 24. The converted sulfur dioxide will travel through the full length of the sampling tubes. Hydrogen sulfide is then determined directly by the analytical instrument 12.

Ammonia concentration is not measured directly, it is measured indirectly by subtraction. Each of the NO/NO$_x$ and NH$_3$ measuring instruments 2 and 11 is the same. Both are set up for alternately measuring two gas samples in rapid succession. The gas actually measured in each instrument is NO. In the NO/NO$_x$ instrument one gas sample is the gas stream directly from the sample inlet. The NO concentration in this sample is measured directly. The other sample is from the same stream, but is passed through a catalytic converter (not shown) which is part of the instrument and which converts all NO$_x$ compounds to NO. The measurements of NO in the two samples are then subtracted to yield a value for the NO$_x$ in the sample.

The amount of ammonia is determined in a generally similar manner. In the NH$_3$ instrument 11, however, one sample passes through a catalytic converter which converts all NO$_x$ compounds to NO. The other sample passes through a catalytic converter which converts ammonia and all the $NO_x$ compounds to NO. The difference between these two readings is the ammonia concentration in the air sample.

It is desirable to use a single instrument for these measurements since instrument drift and calibration are not critical to the accuracy of the measurements. In fact, it is desirable to make all the measurements with individual instruments in an analytical laboratory instead of a plurality of instruments at each location in the closed system.

One of the subjects of interest is rather small gradients of gas concentration between different parts of the system. Separate instruments at various locations would require precise calibration for making the subtractions needed to identify such gradients. A tubing system between the instruments and the remote locations permits such comparisons to be made accurately. The differences can actually be determined with greater precision than the absolute accuracy of the measuring instrument would indicate. This desideratum is not unique to measurement of gas concentrations in the closed ecological system. It is also desirable in a system for measuring spread of noxious gas from a leak or spill, for example, where gradients in concentration indicate the nature of the plume.

Each of the sampling tubes is connected to a sample manifold 22, 23 or 24 by a three-way solenoid valve. The third valve connection for each of these valves is to a small capillary orifice 51. Each of the capillary orifices is, in turn, connected to a vacuum manifold 52. The three-way valves are normally in a position that causes air to be drawn into each sample inlet through the respective sampling tubes to the capillary orifice. The orifice serves to restrict flow to a low rate, but maintains flow through all the sampling tubes for continual purging, and assurance that the air in the sampling tubes has a composition representative of the air at the sample inlet.

The solenoid valves permit disconnecting the sampling tube from the vacuum manifold and connecting it to the respective sample manifold when it is desired to analyze the gas from a particular sample inlet. The analysis may be made promptly upon making such a connection because of the continual drawing of air through the sampling tubes or may be delayed a sufficient time for air to travel from the remote sampling inlet to the analytical laboratory. Prompt measurements can be made since the drift of gas composition is quite slow by comparison with the sampling intervals involved.

Although one embodiment of gas sampling and analysis system and minor variations have been described in detail herein, it will be readily apparent that many modifications can be made. For example, the system is described in the context of air sampling in a closed ecological system. A similar system could be used in many other settings. For example, an array of remote locations may be provided around a chemical plant for monitoring air quality and detecting leaks, and for tracking a plume of leaking chemical if that should occur. The analytical instruments in such an embodiment would be selected for their sensitivity to particular chemicals likely to be present in the vicinity. A large array of remote locations may be maintained on standby for observation of accidental leakage or spills.

In the embodiment of air sampling and analysis system hereinabove described and illustrated, calibration tubes 33 run from the calibration manifold to the remote sampling locations so that calibrations may be made through the sampling tubes. If desired, alternative connections can be made so that the calibration tubes double as sampling tubes. The particular gas analysis instruments described are appropriate for the tests to be run in this one situation. Other instruments would be useful for monitoring other gases or vapors. Many other modifications and variations will be apparent to those skilled in the art and it is therefore to be understood that within the scope of the appended claims, the invention may be practiced otherwise than as specifically described.

## Claims

1. An air analysis system comprising:
   a plurality of air sampling tubes having sampling ends at remote locations;
   at least one air analysis instrument;
   means for continually drawing air through each sampling tube from the remote location to the vicinity of the air analysis instrument; and
   means associated with the sampling tubes and the analysis instrument for selectively disconnecting the means for drawing air from any selected sampling tube and connecting the selected sampling tube with the analysis instrument.

2. An air analysis system as recited in claim 1 wherein the means for selectively disconnecting and connecting comprises a three way valve for alternatively connecting a sampling tube with the means for drawing air or with the analysis instrument.

3. An air analysis system as recited in either of claims 1 or 2 wherein the means for drawing air comprises a vacuum manifold and a flow control orifice connecting each sampling tube and the vacuum manifold.

4. An air analysis system as recited in any of the preceding claims further comprising means connected to a sampling tube at each remote location for drying air drawn into the sampling tube.

5. An air analysis system as recited in any of the preceding claims further comprising means connected to a sampling tube at each remote location for converting ammonia to nitrogen oxide in air drawn into the sampling tube.

6. An air analysis system as recited in any of the preceding claims further comprising means connected to a sampling tube at each remote location for converting hydrogen sulfide to sulfur dioxide in air drawn into the sampling tube.

7. An air analysis system as recited in any of the preceding claims further comprising means for introducing a gas lethal to aerobic organisms into each sampling tube at a remote location.

8. An air analysis system as recited in any of the preceding claims further comprising means for introducing a calibration gas into each sampling tube at a remote location.

9. A method for analyzing air for vapors at remote locations comprising the steps of:
   drawing air into a sampling tube;
   adjacent to the inlet end of the tube, converting a vapor which may not travel the full length of the sampling tube to a gas which will travel the full length of the sampling tube; and
   conveying air containing the converted gas through the tube to an analysis instrument.

10. A method as recited in claim 9 wherein the vapor comprises hydrogen sulfide and the converting step comprises converting the hydrogen sulfide to sulfur dioxide.

11. A method as recited in either of claims 9 or 10 wherein the vapor comprises ammonia and the converting step comprises converting the ammonia to nitrogen oxide.

12. A method as recited in any of claims 9 to 11 comprising the steps of:
   analyzing the air containing the converted gas for the concentration of the converted gas;
   analyzing air for the concentration of the same gas without conversion at the inlet to the sampling tube; and
   subtracting the second concentration from the first concentration for determining the original concentration of the vapor at the inlet to the sampling tube.

13. An air analysis system comprising:
   at least one sample collection tube for collecting air samples at a remote location;
   an air dryer connected to the sample collection tube for drying sampled air;
   catalytic means for converting ammonia in sampled air to NO;
   means for measuring the NO concentration in air from the catalytic means;
   means for measuring the concentration of NO in sampled air without conversion of ammonia; and
   means for determining concentration of ammonia in sampled air as a difference between the first and second measurements.

14. An air analysis system comprising:
   at least one sample collection tube for collecting air samples at a remote location;
   an air dryer connected to the sample collection tube for drying sampled air;
   catalytic means for converting hydrogen sulfide in sampled air to $SO_2$;
   means for measuring the $SO_2$ concentration in air from the catalytic means;
   means for measuring the concentration of $SO_2$ in sampled air without conversion of hydrogen sulfide; and
   means for determining concentration of hydrogen sulfide in sampled air as a difference between the first and second measurements.

## DOCUMENTS CONSIDERED TO BE RELEVANT

EP 91115981.2

| Category | Citation of document with indication, where appropriate, of relevant passages | Relevant to claim | CLASSIFICATION OF THE APPLICATION (Int. Cl.5) |
|---|---|---|---|
| X | US - A - 4 090 392 (SMITH) * Totality * | 1,2,4 | G 01 N 1/26 G 01 N 31/10 |
| Y |  | 3,5, 6,9- 14 |  |
| A |  | 7,8 |  |
| Y | PATENT ABSTRACTS OF JAPAN, unexamined applications, C field, vol. 5, no. 24, February 13, 1981 THE PATENT OFFICE JAPANESE GOVERNMENT page 2 C 43 * Kokai-no. 55-149 111 (MITSUBISHI DENKI) * | 5,9, 11-13 |  |
| Y | SOVIET PATENTS ABSTRACTS, S-X Electrical, week 8921, July 5, 1989 DERWENT PUBLICATIONS LTD., London, S03 * SU-1436-067 (ANALYT INSTR RES) * | 6,10, 14 | TECHNICAL FIELDS SEARCHED (Int. Cl.5) G 01 N |
| Y | US - A - 4 601 211 (WHISTLER) * Abstracts; fig. * | 3 |  |
| A |  | 1,9 |  |

The present search report has been drawn up for all claims

| Place of search | Date of completion of the search | Examiner |
|---|---|---|
| VIENNA | 20-11-1991 | NARDAI |

EPO FORM 1503 03.82 (P0401)